# EUROPÄISCHE PATENTSCHRIFT

(11) **EP 1 140 741 B1**
(45) Veröffentlichungstag und Bekanntmachung des Hinweises auf die Patenterteilung: **31.03.2004**
(21) Anmeldenummer: 99962263.2
(22) Anmeldetag: 21.12.1999
(51) Int. Cl.: C07C 6/04, C07C 11/107, C07C 2/10, C07C 11/02, C07C 29/16, C07C 31/125, C07C 41/03, C07C 43/13, C07F 9/11, C07F 9/09

(54) **VERFAHREN ZUR HERSTELLUNG VON TENSIDALKOHOLEN UND TENSIDALKOHOLETHERN, DIE HERGESTELLTEN PRODUKTE UND IHRE VERWENDUNG**
METHOD FOR PRODUCING SURFACTANT ALCOHOLS AND SURFACTANT ALCOHOL ETHERS, THE RESULTING PRODUCTS AND THEIR USE
PROCEDE DE PRODUCTION D'ALCOOLS TENSIOACTIFS ET D'ETHERS D'ALCOOLS TENSIOACTIFS, PRODUITS AINSI OBTENUS ET LEUR UTILISATION

(30) Priorität: 23.12.1998 DE 19859911
(43) Veröffentlichungstag der Anmeldung: 10.10.2001
(73) Patentinhaber: BASF AKTIENGESELLSCHAFT, 67056 Ludwigshafen (DE)
(72) Erfinder: MAAS, Heiko, D-67105 Schifferstadt (DE); RÖPER, Michael, D-67157 Wachenheim (DE); WALTER, Marc, D-67227 Frankenthal (DE); SCHULZ, Ralf, D-67346 Speyer (DE); TROPSCH, Jürgen, D-67354 Römerberg (DE); JÄGER, Hans-Ulrich, D-67434 Neustadt (DE); SCHWAB, Peter, D-67098 Bad Dürkheim (DE); SCHULZ, Michael, D-67067 Ludwigshafen (DE)
(74) Vertreter: Isenbruck, Günter, Dr.
(86) Internationale Anmeldenummer: PCT/EP1999/010237
(87) Internationale Veröffentlichungsnummer: WO 2000/039058

(56) Entgegenhaltungen:
- DE-A- 4 339 713
- DE-A- 19 604 466
- GB-A- 1 471 481
- US-A- 3 448 163

## Beschreibung

Die vorliegende Erfindung betrifft ein Verfahren zur Herstellung von Tensidalkoholen und Tensidalkoholethem, die sich u.a. sehr gut als Tenside bzw. zur Herstellung von Tensiden eignen. Dabei werden, ausgehend von C₄-Olefin-Strömen durch eine Metathesereaktion Olefine oder Olefingemische hergestellt, die zu einem Olefingemisch mit 10 bis 16 C-Atomen dimerisiert werden, welches weniger als 10 Gew.-% von Verbindungen enthält, die eine Vinylidengruppe aufweisen, die Olefine anschließend zu Tensidalkoholen derivatisiert und diese gegebenenfalls alkoxyliert.
Die Erfindung betrifft ferner die Verwendung der Tensidalkohole und Tensidalkoholether zur Herstellung von Tensiden durch Glycosidierung oder Polyglycosidierung, Sulfatierung oder Phosphatierung.

Fettalkohole mit Kettenlängen von C₈ bis C₁₈ werden zur Herstellung von nichtionischen Tensiden eingesetzt. Diese werden mit Alkylenoxiden zu den entsprechenden Fettalkoholethoxilaten umgesetzt. (Kap. 2.3 in: Kosswig/Stache, "Die Tenside", Carl Hanser Verlag, München Wien (1993)). Dabei beeinflußt die Kettenlänge des Fettalkohols unterschiedliche Tensideigenschaften wie z.B. Netzvermögen, Schaumbildung, Fettlösevermögen, Reinigungskraft.
Fettalkohole mit Kettenlängen von C₈ bis C₁₈ können auch zur Herstellung von anionischen Tensiden, wie Alkylphosphaten und Alkyletherphosphaten genutzt werden. Anstelle von Phosphaten können auch die entsprechenden Sulfate hergestellt werden. (Kap. 2.2 in: Kosswig/Stache, "Die Tenside", Carl Hanser Verlag, München Wien (1993)).

Die DE-A-196 04 466 betrifft wäßrige Zubereitungen enthaltend ein Alkylpolyglycosid und ein Polyethylenglycolderivat der dort angegebenen Formel I.

Der in dem Polyglycosid enthaltene Alkylrest R² (Seite 2, Zeile 55) soll 8 bis 18, vorzugsweise 10 bis 16 C-Atome enthalten, über seinen Verzweigungsgrad finden sich keine direkten Angaben. Aus der auf Seite 3, Zeile 11 gemachten Angabe, dass der Alkylrest sich insbesondere von Fettalkoholen ableiten soll, die durch Hydrierung nativer Fettsäuren erhalten werden, ist zu schließen, dass es sich um weitgehend lineare Alkylreste handeln muss.

Solche Fettalkohole sind aus nativen Quellen zugänglich, z.B. aus Fetten und Ölen, oder aber auf synthetischem Weg durch Aufbau aus Bausteinen mit einer geringeren Zahl an Kohlenstoffatomen. Eine Variante dabei ist die Dimerisierung eines Olefins zu einem Produkt mit doppelter Anzahl von Kohlenstoffatomen und seine Funktionalisierung zu einem Alkohol.
Zur Dimerisierung von Olefinen ist eine Reihe von Verfahren bekannt. So kann die Reaktion an einem heterogenen Kobaltoxid/Kohle-Katalysator durchgeführt werden (DE-A-1 468 334), in Gegenwart von Säuren wie Schwefel- oder Phosphorsäure (FR 964 922), Aluminiumalkyl-katalysiert (WO 97/16398), oder mit einem gelösten Nickelkomplex-Katalysator (US-A-4 069 273). Nach den Angaben der US-A-4 069 273 erhält man bei der Verwendung dieser Nickelkomplex-Katalysatoren - wobei als Komplexbildner 1,5-Cyclooctadien oder 1,1,1,5,5,5 Hexafluorpentan-2,4-dion eingesetzt werden - hochgradig lineare Olefine mit hohem Anteil an Dimerisierungsprodukten.

Die DE-A-43 39 713 (D1) betrifft ein Verfahren zur Oligomerisierung von Olefinen und Katalysatoren, die so optimiert worden sind, dass sie bei diesem Verfahren besonders hohe Anteile der dort erwünschten linearen Reaktionsprodukte liefern.

In den Ausführungsbeispielen 3 bis 5 werden Butan/Buten-Gemische oligomerisicrt, wobei Reaktionsgemische mit 62 bis 78 Gew.-% Octen-Anteilen erhalten werden. Dieses bekannte Verfahren weist keinen Metatheseschritt auf und die einzigen offenbarten Reaktionsprodukte bestehen nicht aus Gemischkomponenten mit 10 bis 16 C-Atomen.

Die US-A-3,448,163 (D3) betrifft ein Verfahren zur Disproportionierung von Olefinen und für dieses Verfahren besonders geeignete Katalysatoren.

Das einzige Ausführungsbeispiel zeigt, dass aus Buten-1 ein Gemisch von Olefinen mit 2 bis 7 C-Atomen, vorwiegend Ethylen und Hexen-3, entsteht. Dieses bekannte Verfahren weist keinen Dimerisierungsschritt auf und das einzige offenbarte Reaktionsprodukt enthält keine Gemischkomponenten mit 10 bis 16 C-Atomen.

Die Funktionalisierung der Olefine zu Alkoholen unter Aufbau des Kohlenstoffgerüstes um ein C-Atom erfolgt zweckmäßigerweise über die Hydroformylierungsreaktion, die ein Gemisch von Aldehyden und Alkoholen liefert, welches nachfolgend zu Alkoholen hydriert werden kann. Jährlich werden weltweit etwa 7 mio Tonnen Produkte mit Hilfe der Hydroformylierung von Olefinen hergestellt. Eine Übersicht über Katalysatoren und Reaktionsbedingungen des Hydroformylierungsverfahrens geben zum Beispiel Beller et al. in Joumal of Molecular Catalysis, A104 (1995), 17-85 oder auch Ullmanns Encyclopedia ofIndustrial Chemistry, Bd. A5 (1986), Seite 217 ff., Seite 333, sowie die diesbezüglichen Literaturverweise.

Die GB-A-1 471 481 (D2) betrifft ein Verfahren zur Hydroformylierung von Olefinen unter Verwendung eines Kobalt enthaltenden Katalysators. Die dort eingesetzten Olefinc sind linear und liefern demgemäß nur wenig verzweigte Oxoalkohole und -aldehyde.

Aus der WO 98/23566 ist es bekannt, dass Sulfate, Alkoxylate, Alkoxysulfate und Karboxylate einer Mischung verzweigter Alkanole (Oxo-Alkohole) gute Oberflächenaktivität in kaltem Wasser zeigen und gute Bio-Abbaubarkeit aufweisen. Die Alkanole der eingesetzten Mischung haben eine Kettenlänge von über 8 Kohlenstoffatomen, sie weisen im Durchschnitt 0,7 bis 3 Verzweigungen auf. Die Alkanolmischung kann, beispielsweise durch Hydroformylierung, hergestellt werden aus Mischungen verzweigter Olefine, die ihrerseits entweder durch Skelettisomerisierung oder durch Dimerisierung interner, linearer Olefine erhalten werden können.
Als Vorteil des Verfahrens wird genannt, daß zur Herstellung des Dimerisierungs-Feeds kein C₃- oder C₄-Olefin-Strom verwendet wird. Daraus forlgt, daß nach dem derzeitigen Stand der Technik die dort der Dimerisierung unterworfenen Olefine aus Ethylen hergestellt worden sein müssen (z.B. SHOP-Verfahren). Da Ethylen für die Tensidherstellung ein relativ teures Ausgangsmaterial ist, sind Ethylen-basierende Verfahren gegenüber Verfahren, die von C₃- und/oder C₄-Olefin-Strömen ausgehen, wirtschaftlich benachteiligt.
Ein weiterer Nachteile dieses bekannten Verfahrens ist die für die Herstellung verzweigter Tensid-Oxo-Alkohole erforderliche Verwendung von Gemischen interner Olefine, die nur durch Isomerisierung von Alphaolefinen zugänglich sind. Solche Verfahren führen immer zu Isomerengemischen, die wegen der unterschiedlichen physikalischen und chemischen Daten der Komponenten verfahrenstechnisch schlechter zu handhaben sind, als Reinsubstanzen. Außerdem ist der zusätzliche Verfahrensschritt der Isomerisierung erforderlich, wodurch das Verfahren einen weiteren Nachteil aufweist.
Die Dimerisierung eines reinen internen Olefins, wie 2-Penten oder 3-Hexen, sowie die weitere Derivatisierung der Dimerisierungsprodukte sind nicht vorbeschrieben.

Die Struktur der Komponenten der Oxo-Alkanolmischung richtet sich nach der Art des Olefingemisches, das der Hydroformylierung unterworfen wurde. Olefingemische, die durch Skelettisomerisierung aus Alphaolefingemischen erhalten wurden, fuhren zu Alkanolen, die überwiegend an den Enden der Hauptkette, d.h. in den Positionen 2 und 3, gerechnet vom jeweiligen Kettenende, verzweigt sind (Seite 56, letzter Absatz). Aus Olefingemischen, die durch Dimerisierung von Olefinen kürzerer Kettenlänge gewonnen wurden, werden nach dem in dieser Druckschrift offenbarten Verfahren Oxoalkohole erhalten, deren Verzweigungen mehr in der Mitte der Haupkette liegen, und zwar, wie die Tabelle IV auf Seite 68 zeigt, weit überwiegend an C4 und weiter entfernten Kohlenstoffatomen, bezogen auf das Hydroxyl-Kohlenstoffatom. An den C2 und C3 Positionen, bezogen auf das Hydroxyl-Kohlenstoffatom, dagegen finden sich weniger als 25% der Verzweigungen (Seiten 28/29 dieses Dokuments).

Die Oberflächenaktiven Endprodukte werden aus den Alkanolmischungen entweder durch Oxydieren der -CH₂OH-Gruppe zur Carboxylgruppe, oder durch Sulfatieren der Alkanole oder ihrer Alkoxylate gewonnen.

Ähnliche Verfahren zur Herstellung von Tensiden werden in der PCT-Patentanmeldung WO 97/38957 und in der EP-A-787 704 beschrieben. Auch bei den dort beschriebenen Verfahren wird ein Alphaolefin zu einem Gemisch vorwiegend vinylidenverzweigter Olefindimerer dimerisiert (WO 97/38957):

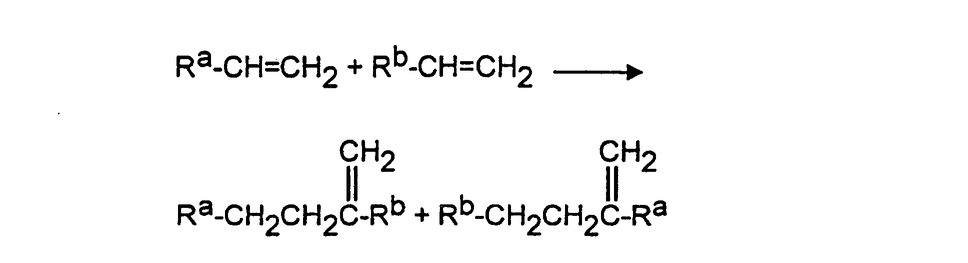

Anschließend werden die Vinylidenverbindungen doppelbindungsisomerisiert, sodaß die Doppelbindung vom Kettenende weiter in die Mitte wandert und anschließend der Hydroformylierung zu einem Oxoalkohol-Gemisch unterworfen. Dieses wird dann weiter, z.B. durch Sulfatierung zu Tensiden umgesetzt. Ein gravierender Nachteil dieses Verfahrens besteht darin, daß es von Alphaolefinen ausgeht. Alphaolefine werden z.B. durch Übergangsmetall-katalysierte Oligomerisierung von Ethylen, Ziegler-Aufbaureaktion, Wachscracken oder Fischer-Tropsch-Verfahren gewonnen und stellen daher relativ teure Ausgangsmaterialien für die Tensidherstellung dar. Ein weiterer erheblicher Nachteil dieses bekannten Tensid-Herstellungsverfahrens besteht darin, daß bei ihm zwischen der Dimerisierung der Alphaolefine und der Hydroformylierung des Dimerisierungsprodukts eine Skelettisomerisierung eingeschaltet werden muß, wenn man zu überwiegend verzweigten Produkten kommen will. Aufgrund der Verwendung eines für die Tensidherstellung relativ teuren Ausgangsmaterials und der Notwendigkeit, einen zusätzlichen Verfahrensschritt, die Isomerisierung, einzuschalten ist dieses bekannte Verfahren wirtschaftlich erheblich benachteiligt.

Es wurde nun überraschenderweise gefunden, daß man zur Herstellung von verzweigten Olefinen und Alkoholen (Oxoalkoholen), die sich zu sehr gut wirksamen Tensiden weiterverarbeiten lassen - im Folgenden als "Tensidalkohole" bezeichnet-, weder auf Alphaolefine noch auf Olefine, die hauptsächlich aus Ethylen hergestellt wurden, angewiesen ist, sondern daß man von preisgünstigen C₄-Olefin-Strömen ausgehen kann, und daß man darüberhinaus den Isomerisierungsschritt vermeiden kann, wenn man nach dem im Folgenden beschriebenen erfindungsgemäßen Verfahren arbeitet.
C₄-Olefin-Ströme sind Gemische, die im wesentlichen, vorzugsweise zu über 80 bis 85 Vol.-%, insbesondere zu über 98 Vol.-% aus Buten-1 und Buten-2 bestehen, und im geringeren Umfang - in der Regel nicht mehr als 15 bis 20 Vol.-% - n-Butan und Isobutan neben Spuren von C₅-Kohlenwasserstoffen enthalten. Diese, in der Fachsprache auch als "Raffinat II" bezeichneten Kohlenwasserstoffgemische entstehen als Nebenprodukt bei der Crackung hochmolekularer Kohlenwasserstoffe, z.B. von Rohöl. Während die bei diesem Prozess entstehenden niedermolekularen Olefine, Ethen und Propen, begehrte Rohstoffe zur Herstellung von Polyethylen und Polypropylen sind und die Kohlenwasserstofffraktionen über C₆ als Kraftstoffe in Verbrennungsmotoren und zu Heizzwecken eingesetzt werden, konnte das Raffinat II, insbesondere dessen C₄-Olefine, bislang nicht in ausreichendem Umfang zu wertvollen Endprodukten weiterverarbeitet werden. Die im Folgenden benutzte Bezeichnung C₄-Olefin-Ströme soll daher auch das als Raffinat II bezeichnete Gasgemisch umfassen.

Das erfindungsgemäße Verfahren eröffnet nun einen verfahrensmäßig sehr günstigen Weg, die anfallenden C₄-Olefin-Ströme zu wertvollen Tensidalkoholen zu verarbeiten, aus denen dann nach verschiedenen an sich bekannten Methoden nichtionische oder anionische Tenside hergestellt werden können.

Ein Gegenstand dieser Erfindung ist ein Verfahren zur Herstellung von Tensidalkoholen und Tensidalkoholethem durch Derivatisierung von Olefinen mit etwa 10 bis 20 C-Atomen oder von Gemischen solcher Olefine und gegebenenfalls anschließende Alkoxylierung dadurch gekennzeichnet, daß man
a) ein C₄-Olefin-Gemisch der Metathese unterwirft,
b) aus dem Metathesegemisch Olefine mit 5 bis 8 C-Atomen abtrennt,
c) die abgetrennten Olefine einzeln oder im Gemisch einer Dimerisierung zu Olefingemischen mit 10 bis 16 C-Atomen unterzieht,
d) das erhaltene Olefingemisch, gegebenenfalls nach einer Fraktionierung, der Derivatisierung zu einem Gemisch von Tensidalkoholen unterwirft und
e) gegebenenfalls die Tensidalkohole alkoxyliert.

Die Grundzüge der im Verfahrensschritt a) eingesetzten Metathese sind beispielsweise in Ullmann's Ecyclopedia of Industrial Chemistry, 5. Aufl., Band A18, S.235/236 beschrieben worden. Weitere Informationen zur Durchführung des Verfahrens können beispielsweise K.J. Ivin, "Olefin Metathesis, Academic Press, London, (1983); Houben-Weyl, E18, 1163-1223; R.L. Banks, Discovery and Development of Olefin Disproportionation, CHEMTECH (1986), February, 112-117, entnommen werden.

Bei der Anwendung der Metathese auf die in den C₄-Oleftn-Strömen enthaltenen Hauptbestandteile Buten-1 und Buten-2 werden in Gegenwart von geeigneten Katalysatoren Olefine mit 5 bis 10 C-Atomen, vorzugsweise mit 5 bis 8 C-Atomen, insbesondere aber Penten-2 und Hexen-3 gebildet

Geeignete Katalysatoren sind vorzugsweise Molybdän-, Wolfram- oder Rheniumverbindungen. Es ist besonders zweckmäßig, die Reaktion heterogenkatalysiert auszuführen, wobei die katalytisch wirksamen Metalle insbesondere in Verbindung mit Trägern aus Al₂O₃ oder SiO₂ eingesetzt werden. Beispiele für derartige Katalysatoren sind MoO₃ oder WO₃ auf SiO₂, oder Re₂O₇ auf Al₂O₃.

Besonders günstig ist es, die Metathese in Gegenwart eines Rheniumkatalysators auszuführen, da in diesem Fall besonders milde Reaktionsbedingungen möglich sind. So kann die Metathese in diesem Fall bei einer Temperatur von 0 bis 50°C und bei niedrigen Drucken von ca. 0,1 bis 0,2 MPa ausgeführt werden.

Bei der Dimerisierung der im Metatheseschritt erhaltenen Olefine oder Olefingemische erhält man Dimerisierungsprodukte, die im Hinblick auf die weitere Verarbeitung auf Tensidalkohole besonders günstige Komponenten und eine besonders vorteilhafte Zusammensetzungen aufweisen, wenn man einen Dimerisierungskatalysator einsetzt, der wenigstens ein Element der VIII. Nebengruppe des periodischen Systems enthält,
und man die Katalysatorzusammensetzung und die Reaktionsbedingungen so wählt, daß ein Dimerengemisch erhalten wird, welches weniger als 10 Gew.-% von Verbindungen enthält, die ein Strukturelement der Formel I (Vinylidengruppe) 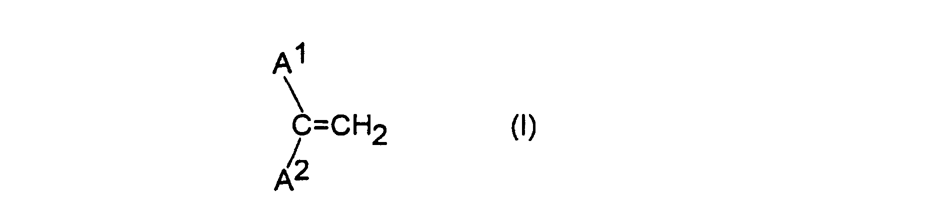 worin A¹ und A² aliphatische Kohlenwasserstoffreste sind,
aufweisen.
Vorzugsweise werden für die Dimerisierung die in dem Metathesierungsprodukt enthaltenen internen, linearen Pentene und Hexene eingesetzt. Besonders bevorzugt ist der Einsatz von 3-Hexen.

Die Dimerisierung kann homogenkatalysiert oder heterogenkatalysiert durchgeführt werden. Bevorzugt ist die heterogene Verfahrensweise, da hierbei einerseits die Katalysatorabtrennung vereinfacht und das Verfahren damit wirtschaftlicher ist, zum anderen werden keine umweltschädlichen Abwässer erzeugt, wie sie gewöhnlich bei der Abtrennung gelöster Katalysatoren, zum Beispiel durch Hydrolyse, anfallen. Ein weiterer Vorteil des heterogenen Verfahrens besteht darin, daß das Dimerisierungsprodukt keine Halogene, insbesondere Chlor oder Fluor, enthält. Homogen lösliche Katalysatoren enthalten im allgemeinen halogenidhaltige Liganden oder sie werden in Kombination mit halogenhaltigen Cokatalysatoren eingesetzt. Aus solchen Katalysatorsystemen kann Halogen in die Dimerisierungsprodukte eingebaut werden, was sowohl die Produktqualität als auch die Weiterverarbeitung, insbesondere die Hydroformylierung zu Tensidalkoholen erheblich beeinträchtigt.

Zur heterogenen Katalyse werden zweckmäßigerweise Kombinationen von Oxiden von Metallen der VIII. Nebengruppe mit Aluminiumoxid auf Trägermaterialien aus Silizium- und Titanoxiden wie sie beispielsweise aus der DE-A-43 39 713 bekannt sind, eingesetzt. Der heterogene Katalysator kann im Festbett - dann vorzugsweise in grobkörniger Form als 1 bis 1,5 mm-Splitt - oder suspendiert (Partikelgröße 0.05 bis 0,5 mm) eingesetzt werden. Die Dimerisierung wird bei heterogener Durchführung zweckmäßigerweise bei Temperaturen von 80 bis 200°C, vorzugsweise von 100 bis 180°C, unter dem bei der Reaktionstemperatur herrschenden Druck, gegebenenfalls auch unter einem Schutzgasüberdruck, im geschlossenen System ausgeführt. Zur Erzielung optimaler Umsätze wird das Reaktionsgemisch mehrfach im Kreis geführt, wobei kontinuierlich ein bestimmter Anteil des zirkulierenden Produkts ausgeschleust und durch Ausgangsmaterial ersetzt wird.

Bei der erfindungsgemäßen Dimerisierung werden Mischungen einfach ungesättigter Kohlenwasserstoffe erhalten, deren Komponenten überwiegend die doppelte Kettenlänge haben wie die Ausgangs-Olefine.
Die Dimerisierungskatalysatoren und die Reaktionsbedingungen werden im Rahmen der obigen Angaben zweckmäßigerweise so gewählt, daß mindestens 80 % der Komponenten des Dimerisierungsgemisches im Bereich von 1/4 bis 3/4, vorzugsweise von 1/3 bis 2/3, der Kettenlänge ihrer Hauptkette eine Verzweigung, oder zwei Verzweigungen an benachbarten C-Atomen, aufweisen.
Sehr charakteristisch für die erfindungsgemäß hergestellten Olefingemische ist ihr hoher Anteil - in der Regel über 75%, insbesondere über 80% - von Komponenten mit Verzweigungen und der geringe Anteil - in der Regel unter 25, insbesondere unter 20% - unverzweigter Olefine. Ein weiteres Charakteristikum ist, daß an den Verzweigungsstellen der Hauptkette überwiegend Gruppen mit (y-4) und (y-5) C-Atomen gebunden sind, wobei y die Kohlenstoffatom-Anzahl des für die Dimerisierung eingesetzten Monomers ist. Der Wert (y-5)=0 bedeutet, daß keine Seitenkette vorhanden ist.

Bei erfindungsgemäß hergestellten C₁₂-Olefingemischen trägt die Hauptkette an den Verzweigungspunkten vorzugsweise Methyl- oder Ethylgruppen.
Die Stellung der Methyl- und Ethylgruppen an der Hauptkette ist ebenfalls charakteristisch: Bei Monosubstitution befinden sich die Methyl- oder Ethylgruppen in der Position P = (n/2)-m der Hauptkette, wobei n die Länge der Hauptkette und m die Kohlenstoffanzahl der Seitengruppen ist, bei Disubstitutionsprodukten befindet sich ein Substituent in der Position P, der andere am benachbarten C-Atom P+1. Die Anteile von Monosubstitutionsprodukten (Einfachverzweigung) am erfindungsgemäß hergestellten Olefingemisch liegen charakteristischerweise insgesamt im Bereich von 40 bis 75 Gew.-%, die Anteile an doppeltverzweigten Komponenten im Bereich von 5 bis 25 Gew.-%.
Es wurde ferner gefunden, daß die Dimerisierungsgemische dann besonders gut weiter zu derivatisieren sind, wenn die Lage der Doppelbindung bestimmte Anforderungen erfüllt. In diesen vorteilhaften Olefingemischen ist die Lage der Doppelbindungen relativ zu den Verzweigungen dadurch charakterisiert, daß das Verhältnis der "aliphatischen" Wasserstoffatome zu "olefinischen" Wasserstoffatomen im Bereich
H_{aliph.} : H_{olefin.} = (2*n-0,5) : 0,5 bis (2*n-1,9) : 1,9 liegt, wobei n die Kohlenstoffatom-Anzahl des bei der Dimerisierung erhaltenen Olefins ist.
(Als "aliphatische" Wasserstoffatome werden solche bezeichnet, die an Kohlenstoffatome gebunden sind, die an keiner C=C-Doppelbindung (Pi-Bindung) beteiligt sind, als "olefinische" Wasserstoffatome solche, die an ein Kohlenstoffatom gebunden ist, das eine Pi-Bindung betätigt.)
Besonders bevorzugt sind Dimerisierungsgemische, bei denen das Verhältnis
H_{aliph.} : H_{olefin.} = (2*n-1,0) : 1 bis (2*n-1,6) : 1,6 ist.

Die nach dem erfindungsgemäßen Verfahren erhältlichen neuen Olefingemische mit den oben genannten Strukturmerkmalen sind ebenfalls ein Gegenstand der vorliegenden Erfindung. Sie stellen wertvolle Zwischenprodukte insbesondere für die im Folgenden beschriebene Herstellung von verzweigten primären Alkoholen und Tensiden dar, können aber auch als Ausgangsmaterialien in anderen, von Olefinen ausgehenden technischen Prozessen eingesetzt werden, insbesondere dann, wenn die Endprodukte eine verbesserte biologische Abbaubarkeit haben sollen.

Sollen die erfindungsgemäßen Olefingemische zur Herstellung von Tensiden dienen, so werden sie zunächst nach an sich bekannten Verfahren zu Tensidalkoholen derivatisiert.
Hierzu bestehen verschiedene Wege, die entweder die direkte oder eine auf Umwegen erfolgende Anlagerung von Wasser (Hydratisierung) an die Doppelbindung oder eine Anlagerung von CO und Wasserstoff (Hydroformylierung) an die C=C-Doppelbindung umfassen.

Die Hydratisierung der aus dem Verfahrensschritt c) hervorgehenden Olefine erfolgt zweckmäßigerweise durch direkte Wasseranlagerung unter Protonenkatalyse. Natürlich ist auch ein Umweg, beispielsweise über die Addition von hochprozentiger Schwefelsäure zu einem Alkanolsulfat und anschließende Verseifung zum Alkanol möglich. Die zweckmäßigere direkte Wasseranlagerung wird in Gegenwart saurer, insbesondere heterogener, Katalysatoren und in der Regel bei möglichst hohem Olefin-Partialdruck und bei möglichst niedrigen Temperaturen durchgeführt. Als Katalysatoren bewähren sich insbesondere Phosphorsäure auf Trägern, wie z.B. SiO₂ oder Celite, oder auch saure Ionenaustauscher. Die Wahl der Bedingungen richtet sich nach der Reaktivität der umzusetzenden Olefine und kann routinemäßig durch Vorversuche ermittelt werden (Lit.: z.B. A.J.Kresge et al. J.Am.Chem.Soc. 93, 4907 (1971); Houben-Weyl Bd. 5/4 (1960), Seiten 102-132 und 535-539). Die Hydratisierung führt in der Regel zu Mischungen von primären und sekundären Alkanolen, in denen die sekundären Alkanole überwiegen.

Für die Herstellung von Tensiden ist es günstiger, von primäre Alkanolen auszugehen. Es daher bevorzugt, die Derivatisierung der aus Schritt c) erhaltenen Olefingemische durch Umsetzung mit Kohlenmonoxid und Wasserstoff in Gegenwart von geeigneten, vorzugsweise kobalt- oder rhodiumhaltigen Katalysatoren zu verzweigten primären Alkoholen zu hydroformylieren.

Ein weiterer bevorzugter Gegenstand der vorliegenden Erfindung ist daher ein Verfahren zur Herstellung von Mischungen primärer Alkanole, die sich u.a. zur Weiterverarbeitung zu Tensiden eignen durch Hydroformylierung von Olefinen, das dadurch gekennzeichnet ist, daß man die oben beschriebenen erfindungsgemäßen Olefingemische als Ausgangsmaterial einsetzt.
Eine gute Übersicht über das Verfahren der Hydroformylierung mit zahlreichen weiteren. Literaturhinweisen findet sich beispielsweise in dem umfangreichen Aufsatz von Beller et al. in Journal of Molecular Catalysis, A104 (1995) 17-85 oder in
Ullmanns Encyclopedia of Industrial Chemistry, Bd.A5 (1986), Seite 217 ff., Seite 333, sowie die diesbezüglichen Literaturverweise.
Die dort gegebenen umfassenden Informationen ermöglichen es dem Fachmann, auch die erfindungsgemäßen verzweigten Olefine zu hydroformylieren. Bei dieser Reaktion wird CO und Wasserstoff an olefinische Doppelbindungen angelagert, wobei gemäß dem folgenden Reaktionsschema Mischungen aus Aldehyden und Alkanolen erhalten werden:

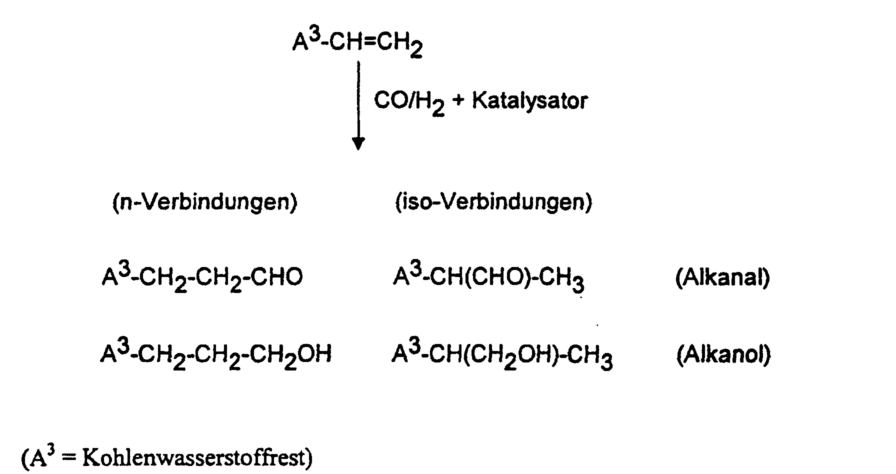

Das Molverhälnis von n- und iso-Verbindungen im Reaktionsgemisch liegt je nach den gewählten Verfahrensbedingungen der Hydroformylierung und dem eingesetzten Katalysator in der Regel im Bereich von 1:1 bis 20:1. Die Hydroformylierung wird normalerweise im Temperaturbereich von 90 bis 200° und bei einem CO/H₂-Druck von 2,5 bis 35 MPa (25 bis 350 bar) ausgeführt. Das Mischungsverhältnis von Kohlenmonoxid zu Wasserstoff richtet sich danach, ob vorzugsweise Alkanale oder Alkanole erzeugt werden sollen. Man arbeitet zweckmäßigerweise im Bereich CO:H₂
von 10:1 bis 1:10, vorzugsweise 3:1 bis 1:3, wobei man zur Herstellung von Alkanalen den Bereich der niedrigen Wasserstoffpartialdrucke, zur Herstellung von Alkanolen den Bereich der hohen Wasserstoffpartialdrucke, z.B. CO:H₂ = 1:2, wählt.
Als Katalysatoren eignen sich vor allem Metallverbindungen der allgemeinen Formel HM(CO)₄ oder M₂(CO)₈, wobei M ein Metallatom, vorzugsweise ein Kobalt-, Rhodium- oder Rutheniumatom ist.
Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für einen Liganden, der ein Phosphin, Phosphit, Amin, Pyridin oder jede andere Donorverbindung, auch in polymerer Form, sein kann, und q, x, y und z für ganze Zahlen, abhängig von der Wertigkeit und Art des Metalls sowie der Bindigkeit des Liganden L, stehen, wobei q auch 0 sein kann.
Bei dem Metall M handelt es sich vorzugsweise um Kobalt, Ruthenium, Rhodium, Palladium, Platin, Osmium oder Iridium und insbesondere um Kobalt, Rhodium oder Ruthenium.
Geeignete Rhodiumverbindungen oder Komplexe sind z.B. Rhodium(II)- und Rhodium(III)-salze, wie Rhodium(III)-chlorid, Rhodium(III)-nitrat, Rhodium(III)-sulfat, Kalium-Rhodium-sulfat, Rhodium(II) bzw. Rhodium(III)-caboxylat, Rhodium(II)- und Rhodium(III)-acetat, Rhodium(III)-oxid, Salze der Rhodium(III)-säure, wie z.B. Trisammonium-hexachlororhodat-(III). Weiterhin eignen sich Rhodiumkomplexe wie Rhodiumbiscarbonyl-acetylacetonat, Acetylacetonato-bisethylenRhodium-(I). Vorzugsweise werden Rhodiumbiscarbonyl-acetylacetonat oder Rhodiumacetat eingesetzt.

Geeignete Kobaltverbindungen sind zum Beispiel Kobalt(II)-chlorid, Kobalt(II)-sulfat, Kobalt(II)-carbonat, Kobalt(II)-nitrat, deren Amin- oder Hydratkomplexe, Kobaltcarbocylate wie Kobaltacetat, Kobaltethylhexanoat, Kobaltnaphthanoat, sowie der Kobaltcaprolactamat-Komplex. Auch hier können die Carbonylkomplexe des Kobalts wie Dikobaltoctocarbonyl, Tetrakobaltdodecacarbonyl und Hexakobalthexadecacarbonyl eingesetzt werden. Die genannten Verbindungen des Kobalts, Rhodiums und Rutheniums sind im Prinzip bekannt und in der Literatur hinreichend beschrieben, oder sie können vom Fachmann analog zu den bereits bekannten Verbindungen hergestellt werden.

Die Hydroformylierung kann unter Zusatz von inerten Lösungs- oder Verdünnungsmitteln oder ohne solchen Zusatz durchgeführt werden. Geeignete inerte Zusätze sind beispielsweise Aceton, Methyl-ethylketon, Cyclohexanon, Toluol, Xylol, Chlorbenzol, Methylenchlorid, Hexan, Petrolether, Acetonitril sowie die hochsiedenden Anteile aus der Hydroformylierung der Dimerisierungsprodukte.

Sofern das erhaltene Hydroformylierungsprodukt einen zu hohen Aldehydgehalt aufweist, kann dieser auf einfache Weise durch eine Hydrierung, zum Beispiel mit Wasserstoff in Gegenwart von Raney-Nickel oder unter Verwendung anderer für Hydrierungsreaktionen bekannter, insbesondere Kupfer, Zink, Kobalt, Nickel, Molybdän, Zirkon oder Titan enthaltender Katalysatoren, beseitigt werden. Dabei werden die Aldehydanteile weitgehend zu Alkanolen hydriert. Eine praktisch restlose Beseitigung von Aldehydanteilen im Reaktionsgemisch läßt sich gewünschtenfalls durch Nachhydrierung, beispielsweise unter besonders schonenden und ökonomischen Bedingungen mit einem Alkaliborhydrid, erreichen.

Die durch Hydroformylierung der erfindungsgemäßen Olefingemische herstellbaren Gemische verzweigter primärer Alkanole sind ebenfalls ein Gegenstand der vorliegenden Erfindung.

Aus den erfindungsgemäßen Alkanolen können auf unterschiedliche Weise nichtionische oder anionische Tenside hergestellt werden.

Nichtionische Tenside werden erhalten durch Umsetzung der Alkanole mit 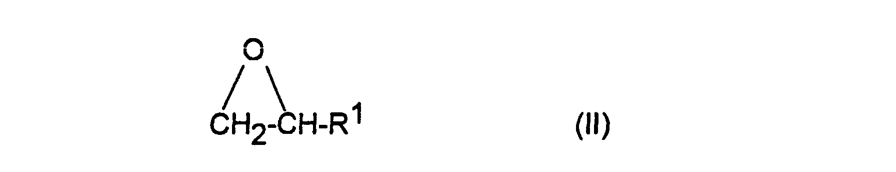 Alkylenoxiden (Alkoxylierung) der Formel II
worin R¹ Wasserstoff oder ein geradkettiger oder verzweigter aliphatischer Rest der Formel CₙH₂ₙ₊₁ ist und n für eine Zahl von 1 bis 16, vorzugsweise von 1 bis 8 steht. Insbesondere bedeutet R¹ Wasserstoff, Methyl oder Ethyl.
Die erfindungsgemäßen Alkanole können mit einer einzigen Alkylenoxid-Species oder mit mehreren verschiedenen umgesetzt werden. Bei der Umsetzung der Alkanole mit den Alkylenoxiden entstehen Verbindungen, die wiederum eine OH-Gruppe tragen und daher erneut mit einem Alkylenoxidmolekül reagieren können.
Es werden daher je nach dem Molverhältnis von Alkanol zu Alkylenoxid Reaktionsprodukte erhalten, die mehr oder weniger lange Polyetherketten aufweisen. Die Polyetherketten können 1 bis ca. 200 Alkylenoxid-Baugruppen enthalten. Bevorzugt sind Verbindungen, deren Polyetherketten 1 bis 10 Alkylenoxid-Baugruppen enthalten.
Die Ketten können aus gleichen Kettengliedern bestehen, oder sie können verschiedene Akylenoxid-Baugruppen aufweisen, die sich bezüglich ihres Restes R¹ voneinander unterscheiden. Diese unterschiedlichen Baugruppen können innnerhalb der Kette in statistischer Verteilung oder in Form von Blöcken vorliegen.

Das folgende Reaktionsschema soll die Alkoxylierung der erfindungsgemäßen Alkanole am Beispiel einer Umsetzung mit zwei verschiedenen Alkylenoxiden, die in unterschiedlichen Mol-Mengen x und y eingesetzt werden, veranschaulichen.

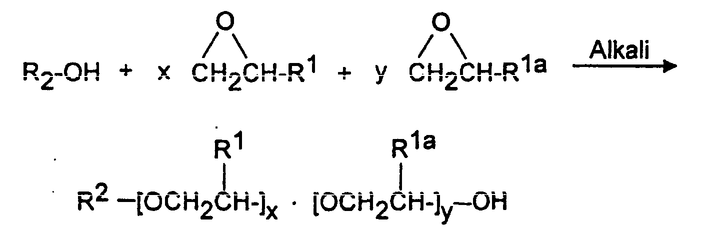

R¹ und R^{1a} sind verschiedene Reste im Rahmen der oben für R¹ gegebenen Definitionen und R²-OH ist ein erfindungsgemäßes verzweigtes Alkanol.
Vorzugsweise wird die Alkoxylierung durch starke Basen katalysiert, die zweckmäßigerweise in Form eines Alkalihydroxids oder Erdalkalihydroxids, in der Regel in einer Menge von 0,1 bis 1 Gew.-% bezogen auf die Menge des Alkanols R²-OH, zugesetzt werden. (Vergl. G.Gee et al., J. Chem. Soc. (1961), S. 1345; B. Wojtech, Makromol. Chem. **66**, (1966), S.180)
Auch eine saure Katalyse der Additionsreaktion ist möglich. Neben Bronstedsäuren eignen sich auch Lewissäuren wie z.B. AlCl₃ oder BF₃. (Vergl. P.H.Plesch, The Chemistry of Cationic Polymerization, Pergamon Press, New York (1963).

Die Additionsreaktion wird bei Temperaturen von etwa 120 bis etwa 220°C, vorzugsweise von 140 bis 160°C, im geschlossenen Gefäß ausgeführt. Das Alkylenoxid oder die Mischung verschiedener Alkylenoxide wird der Mischung aus erfindungsgemäßem Alkanolgemisch und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.
Wird ein Alkylenoxidgemisch eingesetzt, so werden Polyetherketten gebildet in denen die verschiedenen Alkylenoxidbausteine praktisch statistisch verteilt sind. Variationen in der Verteilung der Bausteine längs der Polyetherkette ergeben sich aufgrund unterschiedlicher Reaktionsgeschwindigkeiten der Komponenten und können auch willkürlich durch kontinuierliche Zufuhr einer Alkylenoxidmischung programmgesteuerter Zusammensetzung erreicht werden. Werden die verschiedenen Alkylenoxide nacheinander zur Reaktion gebracht so erhält man Polyetherketten, mit blockartiger Verteilung der Alkylenoxid-Bausteine.
Die Länge der Polyetherketten schwankt innerhalb des Reaktionsprodukts statistisch um einen Mittelwert, der im wesentlichen dem sich aus der Zusatzmenge ergebenden stöchiometrischen Wert.

Die ausgehend von erfindungsgemäßen Olefingemischen und Alkanolgemischen herstellbaren Alkoxylate stellen ebenfalls einen Gegenstand der vorliegenden Erfindung dar. Sie zeigen eine sehr gute Oberflächenaktivität und können daher als neutrale Tenside in vielen Anwendungsbereichen eingesetzt werden.

Ausgehend von den erfindungsgemäßen Alkanolgemischen können auch oberflächenaktive Glycoside und Polyglycoside (Oligoglycoside) hergestellt werden. Auch diese Substanzen zeigen sehr gute Tensideigenschaften. Sie werden erhalten durch ein- oder mehrfache Umsetzung (Glycosidierung bzw. Polyglycosidierung) der erfindungsgemäßen Alkanolgemische mit Mono-, Di- oder Poly-sacchariden unter Ausschluß von Wasser unter Säurekatalyse. Geeignete Säuren sind beispielsweise HCl oder H₂SO₄. In der Regel werden hierbei Oligoglycoside mit statistischer Kettenlängenverteilung erhalten, wobei der durchschnittliche Oligomerisierungsgrad bei 1 bis 3 Sacharid-Resten liegt.
Bei einer anderen Standardsynthese wird das Saccharid zunächst unter Säurekatalyse mit einem niedermolekularen Alkanol, z.B. Butanol, zum Butanolglycosid acetalisiert. Diese Reaktion kann auch mit wäßrigen Lösungen des Saccharids ausgeführt werden. Das Niederalkanolglycosid, beispielsweise das Butanolglycosid, wird dann mit den erfindungsgemäßen Alkanolgemischen zu den gewünschten erfindungsgemäßen Glycosiden umgesetzt. Überschüssige lang- und kurzkettige Alkanole können nach Neutralisieren des sauren Katalysators aus dem Gleichgewichtsgemisch, z.B. durch Abdestillieren im Vakuum, entfernt werden.

Eine weitere Standardmethode verläuft über die O-Acetylverbindungen der Saccharide. Diese werden mit Halogenwasserstoff, der vorzugsweise in Eisessig gelöst ist, in die entsprechenden O-Acetyl-halosaccharide überführt, die in Gegenwart von Säure bindenden Agenzien mit den Alkanolen zu den acetylierten Glycosiden reagieren.
Bevorzugt für die Glycosidierung der erfindungsgemäßen Alkanolgemische sind Monosaccharide, und zwar sowohl die Hexosen wie Glucose, Fructose, Galactose, Mannose, als auch Pentosen wie Arabinose, Xylose oder Ribose. Besonders bevorzugt zur Glycosidierung der erfindungsgemäßen Alkanolgemische ist die Glucose. Selbstverständlich können auch Gemische der genannten Sacharide für die Glycosidierung eingesetzt werden. Es werden dann je nach den Reaktionsbedingungen Glycosiede mit statistisch verteilten Zuckerresten erhalten.
Die Glycosidierung kann auch mehrfach erfolgen, sodaß an die Hydroxylgruppen der Alkanole Polyglycosidketten angelagert werden. Bei einer Polyglycosidierung und Einsatz verschiedener Saccharide können die Saccharid-Bausteine innerhalb der Kette statistisch verteilt sein oder Blöcke gleicher Baugruppen bilden.
Je nach der gewählten Reaktionstemperatur können Furanose- oder Pyranosestrukturen erhalten werden. Zur Verbesserung der Löslichkeitsverhältnisse kann die Reaktion auch in geeigneten Lösungs- oder Verdünnungsmitteln ausgeführt werden.

Standardverfahren und geeignete Reaktionsbedingungen sind in verschiedenen Publikationen beschrieben worden, beispielsweise in "Ullmanns Encyclopedia of Industrial Chemistry", 5.Auflage Bd. A25 (1994), Seiten 792-793 und in den dort angegebenen Literaturverweisen, von K.Igarashi, Adv. Carbohydr. Chem. Biochem. **34**, (1977), S. 243-283, von Wulff und Röhle, Angew. Chem. **86**, (1974), S.173-187, oder bei Krauch und Kunz, Reaktionen der organischen Chemie, S. 405-408, Hüthig, Heidelberg, (1976).
Die ausgehend von erfindungsgemäßen Olefingemischen und Alkanolgemischen herstellbaren Glycoside und Polyglycoside (Oligoglycoside) stellen ebenfalls einen Gegenstand der vorliegenden Erfindung dar.

Sowohl die erfindungsgemäßen Alkanolgemische als auch die daraus hergestellten Polyether können in anionische Tenside überführt werden indem man sie in an sich bekannter Weise mit Schwefelsäure oder Schwefelsäurederivaten zu sauren Alkylsulfaten oder Alkylethersulfaten oder mit Phosphorsäure oder ihren Derivaten zu sauren Alkylphosphaten bzw. Alkyletherphosphaten verestert (sulfatiert).
Sulfatierungsreaktionen von Alkoholen sind bereits beschrieben worden z.B. in US-A-3 462 525, 3 420 875 oder 3 524 864. Details zur Durchführung dieser Reaktion finden sich auch in "Ullmanns Encyclopedia of Industrial Chemistry", 5.Auflage Bd. A25 (1994), Seiten 779-783 und in den dort angegebenen Literaturverweisen.
Wird Schwefelsäure selbst zur Veresterung eingesetzt, so verwendet man zweckmäßigerweise eine 75 bis 100 gew.-%ige, vorzugsweise 85 bis 98 gew.-%ige Säure (sog. "konzentrierte Schwefelsäure" oder "Monohydrat"). Die Veresterung kann in einem Lösungs- oder Verdünnungsmittel vorgenommen werden, wenn es für die Steuerung der Reaktion, z.B. der Wärmeentwicklung erwünscht ist. In der Regel wird der alkoholische Reaktant vorgelegt, und das Sulfatierungsmittel unter ständigem Durchmischen allmählich zugefügt. Wenn eine vollständige Veresterung der Alkoholkomponente gewünscht wird verwendet man das Sulfatierungsmittel und den Alkanol im Molverhältnis von 1:1 bis 1:1,5, vorzugsweise von 1:1 bis 1:1,2. Geringere Mengen von Sulfatierungsmittel können vorteilhaft sein, wenn Mischungen von erfindungsgemäßen Alkanolalkoxylaten eingesetzt und Kombinationen von Neutralen und anionischen Tensiden hergestellt werden sollen. Die Veresterung wird normalerweise bei Temperaturen von Zimmertemperatur bis 85°C, vorzugsweise im Bereich von 45 bis 75°C, ausgeführt.
Gegebenenfalls kann eszweckmäßig sein, die Veresterung in einem niedrig siedenden, mit Wasser nicht mischbaren Lösungs- und Verdünnungsmittel bei dessen Siedepunkt auszuführen, wobei das bei der Veresterung entstehende Wasser azeotrop abdestilliert wird.
Anstelle von Schwefelsäure der oben angegebenen Konzentration kann zur Sulfatierung der erfindungsgemäßen Alkanolgemische auch beispielsweise Schwefeltrioxid, Schwefeltrioxid-Komplexen, Lösungen von Schwefeltrioxid in Schwefelsäure ("Oleum"), Chlorsulfonsäure, Sulfurylchlorid oder auch Amidosulfosäure eingesetzt werden. Die Reaktionsbedingungen sind dann zweckentsprechend anzupassen.

Wird Schwefeltrioxid als Sulfatierungsmittel eingesetzt so kann die Reaktion auch vorteilhafterweise in einem Fallfilmreaktor im Gegenstrom, gewünschtenfalls auch kontinuierlich, ausgeführt werden.
Die Ansätze werden nach der Veresterung durch Alkalizusatz neutralisiert und, ggf. nach Entfernung überschüssigen Alkalisulfates und eventuell vorhandener Lösungsmittel, aufgearbeitet.
Die durch Sulfatierung erfindungsgemäßer Alkanole und Alkanolether und deren Gemische erhaltenen sauren Alkanolsulfate und Alkanolethersulfate und deren Salze sind ebenfalls ein Gegenstand der vorliegenden Erfindung.

In analoger Weise können erfindungsgemäße Alkanole und Alkanolether und deren Gemische mit Posphatierungsagenzien auch zu sauren Phosphorsäureestem umgesetzt (phosphatiert) werden.
Als Posphatierungsagenzien eignen sich vornehmlich Phosphorsäure, Polyphosphorsäure und Phosphorpentoxid, aber auch POCl₃, wenn anschließend eine Hydrolyse der verbleibenden Säurechloridfunktionen vorgenommen wird. Die Phosphatierung von Alkoholen ist beispielsweise in Synthesis 1985, Seiten 449 bis 488 beschrieben worden.

Auch die durch Phosphatierung erfindungsgemäßer Alkanole und Alkanolether und deren Gemische erhaltenen sauren Alkanolphosphate und Alkanoletherphosphate sind ein Gegenstand der vorliegenden Erfindung.

Schließlich ist auch die Verwendung der ausgehend von den erfindungsgemäßen Olefingemischen herstellbaren Alkanolethergemische, Alkanolglycoside sowie die sauren Sulfate und Phosphate der Alkanolgemische und der Alkanolethergemische als Tenside ein Gegenstand der vorliegenden Erfindung.

Die folgenden Ausführungsbeispiele veranschaulichen die Herstellung und Verwendung der erfindungsgemäßen Tenside.

### Beispiel 1

### Herstellung von C₅/C₆-Olefinen aus C₄-Olefin-strömen durch Metathese.

Eine butadienfreie C₄-Fraktion mit einem Gesamtbutengehalt von 84,2 Gew.-% und einem Molverhältnis 1-Buten : 2-Buten von 1,06 ("Raffinat II") wird bei 40 °C und 10 bar kontinuierlich durch einen mit Re₂O₇/Al₂O₃-Heterogenkontakt beschickten Rohrreaktor geleitet. Die Katalysatorbelastung wird auf 4500 kg/(m2*h) eingestellt. Der Reaktoraustrag wird destillativ getrennt und enthält folgende Komponenten (Angaben in Massenprozent):
Ethen: 1,15 %, Propen: 18,9 %, Butan: 15,8 %, 2-Buten: 19,7 %, 1-Buten: 13,3 %, i-Buten: 1,00 %, 2-Penten: 19,4 %, Methylbuten: 0,45 %, 3-Hexen 10,3 %.

### Beispiele 2A und 2B: Heterogenkatalysierte Dimerisierung von 3-Hexen.

### 2A. Festbettverfahren.

Ein isotherm beheizbarer Reaktor mit 16 mm Durchmesser wurde mit 100 ml eines Katalysators der folgenden Zusammensetzung befüllt:
50 Gew.-% NiO, 34 Gew.-% SiO₂, 13 Gew.-% TiO₂, 3 Gew.-% Al₂O₃ (gemäß DE-A-43 39 713), 24 Stdn. bei 160°C in N₂ konditioniert, eingesetzt als 1 bis 1,5 mm Splitt.
Es wurden 5 Versuche gemacht, wobei durch das festgelegte Katalysatorbett 3-Hexen (99,9 gew.-%ig, 0,1 Gew.-% C₇ bis C₁₁-Fraktionen) in einer auf das Reaktorvolumen bezogenen Rate (WHSV) von 0,25 kg/l*h geleitet und in einer Rate von 24 bis 28 g/h ausgeschleust wurde. Variiert wurde bei den einzelnen Versuchen die Reaktionstemperatur oder die Betriebsdauer des Versuchs.
Die folgende Tabelle 1 zeigt die Versuchsbedingungen der fünf Versuche und die dabei erhaltenen Ergebnisse.

**Table 1.**

| Verfahrensbedingungen und Ergebnisse beim Festbettverfahren. | | | | | | |
|---|---|---|---|---|---|---|
| Reaktionsbedingungen | | | | | | |
| Temperatur [°C] | 100 | 120 | 140 | 160 | 160 | C₁₂-Destillat |
| Druck [bar] | 20 | 20 | 20 | 25 | 25 | |
| Betriebsstunden | 12 | 19 | 36 | 60 | 107 | |
| Flüssiganfall [g/h] | 24 | 27 | 27 | 28 | 27 | |
| Zusammensetzung Gew.-% | | | | | | |
| C₆ | 68,5 | 52,7 | 43,6 | 57,0 | 73,2 | 0,1 |
| C₇-C11 | 0,2 | 0,2 | 0,3 | 0,2 | 0,2 | - |
| C₁₂ | 25,9 | 38,6 | 44,0 | 35,6 | 23,6 | 99,9 |
| > C₁₂ | 5,4 | 8,5 | 12,1 | 7,2 | 3,0 | - |
| Umsatz | 31,4 | 47,2 | 56,4 | 42,9 | 26,7 | - |
| C₁₂-Selektivität [Gew,-%] | 82,5 | 81,8 | 78,2 | 83,0 | 88,4 | - |
| S-Gehalt im Flüssiganfall [ppm] | - | - | - | - | - | - |

Das ausgeschleuste Produkt wurde fraktioniert destilliert und eine Bestimmung der Gerüstisomeren der C₁₂-Fraktion durchgeführt. Die Analyse ergab 14,2 Gew.-% n-Dodecene, 31,8 Gew.-% 5-Methylundecene, 29,1 Gew.-% 4-Ethyldecene, 6,6 Gew.-% 5,6-Dimethyldecene, 9,3 Gew.-% 4-Methyl-5-ethylnonene und 3,7 Gew.-% Diethyloctene.

### B. Suspensionsverfahren (Fließbettverfahren)

Ein isotherm beheizbaren Reaktor mit 20 mm Durchmesser und einem Volumen von 157 ml wurde mit 30 g eines Katalysators der folgenden Zusammensetzung befüllt:
50 Gew.-% NiO, 34 Gew.-% SiO₂, 13 Gew.-% TiO₂, 3 Gew.-% Al₂O₃ (gemäß DE-A-43 39 713), 24 Stdn. bei 160°C in N₂ konditioniert, eingesetzt als 0,05 bis 0,5 mm Sprühgut.
Es wurden 6 Versuche gemacht wobei durch das Katalysator-Fließbett von unten her 3-Hexen (99,9 gew.-%ig, 0,1 Gew.-% C₇ bis C₁₁-Fraktionen) in einer auf das Reaktorvolumen bezogenen Rate von 0,25 kg/l*h geleitet wurde. Das aus dem Reaktor austretende Reaktionsprodukt wurde großteils rückgeführt (Rückführung:Feedmenge zwischen ca.45 und 60 variiert). Variiert wurde bei den einzelnen Versuchen auch die Reaktionstemperatur, die Feedmenge, die Kreislaufströmung, die Rückführrate und die WHSV des Versuchs. Die Versuchsdauer betrug 8 Stunden.
Die folgenden Tabellen 2A und 2B zeigen die Versuchsbedingungen der sechs Versuche und die dabei erhaltenen Ergebnisse.

### Tabellen 2

Versuchsbedingungen und Ergebnisse beim Suspensionsverfahren.

**Table 2A:**

| Versuchsbedingungen | | | | | | | |
|---|---|---|---|---|---|---|---|
| Versuch Nr. | Temperatur [°C] | Druck [bar] | Feed [g/h] | Kreislauf [g/h] | Rückführrate [X:1] | WHSV | Betriebsdauer [h] |
| 1 | 130 | 11,0 | 20 | 1200 | 60,0 | 0,127 | 8 |
| 2 | 130 | 11,0 | 23 | 1200 | 52,2 | 0,146 | 8 |
| 3 | 130 | 12,0 | 21 | 1100 | 52,4 | 0,134 | 8 |
| 4 | 130 | 12,2 | 24 | 1100 | 45,8 | 0,153 | 8 |
| 5 | 140 | 13,4 | 23 | 1180 | 51,3 | 0,146 | 8 |
| 6 | 150 | 14,1 | 22 | 1200 | 54,5 | 0,140 | 8 |

**Table 2B:**

| Zusammensetzung des Reaktionsaustrags | | | | | | | |
|---|---|---|---|---|---|---|---|
| Versuch Nr. | % C₆ | % >C₆ | % C₁₂ | % C₁₈ | % C₂₄ | % Umsatz | % C₁₂-Selektivität |
| 1 | 83,9 | 0,5 | 14,3 | 1,1 | 0,2 | 16,1 | 88,82 |
| 2 | 80,5 | 0,5 | 16,9 | 1,8 | 0,3 | 19,5 | 86,67 |
| 3 | 80,3 | 0,4 | 17,0 | 1,9 | 0,3 | 19,7 | 86,29 |
| 4 | 81,6 | 0,5 | 15,5 | 2,0 | 0,3 | 18,4 | 84,24 |
| 5 | 75,9 | 0,5 | 20,4 | 2,6 | 0,5 | 24,1 | 84,65 |
| 6 | 71,1 | 0,6 | 24,0 | 3,5 | 0,7 | 28,9 | 83,04 |

Das ausgeschleuste Produkt wurde fraktioniert destilliert und eine Bestimmung der Gerüstisomeren der C₁₂-Fraktion durchgeführt. Die Analyse ergab 14 Gew.-% n-Dodecene, 32 Gew.-% 5-Methylundecene, 29 Gew.-% 4-Ethyldecene, 7 Gew.-% 5,6-Dimethyldecene, 9 Gew.-% 4-Methyl-5-ethylnonene und 4 Gew.-% Diethyloctene.

### Beispiel 3, Hydroformylierung der erfindungsgemäßen Dodecen-Mischung.

750 g des gemäß Beispiel 2B hergestellten Dodecengemisches werden mit 3,0 g Co₂(CO)₈ bei 185°C und 280 bar CO/H₂ (Vol.-Verhältnis = 1:1,5) unter Zusatz von 75 g H₂O in einem 2,5 1 Hubrührautoklaven 5 Stunden hydroformyliert. Der Reaktionsaustrag wird mit 10 gew.-%iger Essigsäure unter Lufteinleitung bei 90°C oxidativ entkobaltet. Das Oxoprodukt wird unter Zusatz von 10 Gew.-% Wasser in einem 2,5 l Hubrührautoklaven mit 50 g Raney-Nickel bei 125°C und 280 bar Wasserstoffdruck 10 Stunden hydriert. Der Reaktionsaustrag wird fraktioniert destillirt.
450 g einer auf diese Weise hergestellten Tridecanolfraktion werden mit 3,5 g NaBH₄ nachhydriert.
Die OH-Zahl des erhaltenen Tridecanols beträgt 277 mg KOH/g.
¹H-NMR-spektroskopisch wurde ein mittlerer Verzweigungsgrad von 2,3 Methylgruppen/Molekül bestimmt, entsprechend einem Verzweigungsgrad von 1,3.

### Beispiel 3A, Hydroformylierung einer erfindungsgemäßen Dodecenmischung.

2,12 kg des gemäß Beispiel 2A hergestellten Dodecengemisches werden mir 8 g Co₂(CO)₈ bei 185°C und 280 bar CO/H₂ (Volumenverhältnis 1:1) unter Zusatz von 210 g Wasser in einem 5-l Drehrührautoklaven 5 Stunden hydroformyliert. Der Reaktionsaustrag wird mit 10 gew.-%iger Essigsäure unter Lufteinleitung bei 90°C oxidativ entkobaltet. Das erhaltene Oxoprodukt wird in einem 5-l Rohrreaktor in Rieselfahrweise an einem Co/Mo-Festbettkatalysator bei 175°C und 280 bar Wasserstoffdruck unter Zusatz von 10 Gew.-% Wasser hydriert. Das Alkoholgemisch wird destillativ aufgearbeitet. Das erhaltene Tridecanol hat eine OH-Zahl von 279 mg KOH/g; ¹H-NMR-spektroskopisch wird ein mittlerer Verzweigungsgrad von 1,53 gemessen.

### Beispiel 3B, Hydroformylierung einer erfindungsgemäßen Dodecenmischung.

50 mg Rhodiumbiscarbonyl-acetylacetonat, 4,5 g eines Polyethylenimins der Molmasse M_{w} = 460000, bei dem 60 % aller Stickstoffatome mit Laurinsäure acyliert sind, 800 g eines gemäß Beispiel 2A hergestellten Dodecengemisches und 196 g Toluol werden in einem 2,5-l Hubrührautoklaven unter einem Druck von 280 bar CO/H₂ (Volumenverhältnis 1:1) 7 Stunden auf 150°C erhitzt. Dann wird der Autoklav abgekühlt, entspannt und entleert.Durch Gaschromatographie des erhaltenen Reaktionsprodukts wird ein Umsatz des Olefins von 93 % festgestellt. Das erhaltene Oxoprodukt wird in einem 2,5-l Rohrreaktor in Rieselfahrweise an einem Co/Mo-Festbettkatalysator bei 175°C und 280 bar Wasserstoffdruck unter Zusatz von 10 Gew.-% Wasser hydriert und das entstandene Alkoholgemisch destillativ aufgearbeitet. Das erhaltene Tridecanol hat eine OH-Zahl von 279 mg KOH/g; ¹H-NMR-spektroskopisch wird ein mittlerer Verzweigungsgrad von 1,63 gemessen.

### Beispiel 3C, Hydroformylierung einer erfindungsgemäßen Dodecenmischung.

50 mg Rhodiumbiscarbonyl-acetylacetonat, 4,5 g eines Polyethylenimins der Molmasse M_{w} = 460000, bei dem 60 % aller Stickstoffatome mit Laurinsäure acyliert sind, 800 g eines gemäß Beispiel 2A hergestellten Dodecengemisches und 196 g Toluol werden in einem 2,5-l Hubrührautoklaven unter einem Druck von 280 bar CO/H₂ (Volumenverhältnis 1:1) 7 Stunden auf 160°C erhitzt. Dann wird der Autoklav abgekühlt, entspannt und entleert.Durch Gaschromatographie des erhaltenen Reaktionsprodukts wird ein Umsatz des Olefins von 94 % festgestellt. Das erhaltene Oxoprodukt wird in einem 2,5-l Rohrreaktor in Rieselfahrweise an einem Co/Mo-Festbettkatalysator bei 175°C und 280 bar Wasserstoffdruck unter Zusatz von 10 Gew.-% Wasser hydriert und das entstandene Alkoholgemisch destillativ aufgearbeitet. Das erhaltene Tridecanol hat eine OH-Zahl von 279 mg KOH/g; ¹H-NMR-spektroskopisch wird ein mittlerer Verzweigungsgrad von 1,69 gemessen.

### Beispiele 4A und 4B, Herstellung von Fettalkoholethoxilaten.

### A. Fettalkoholethoxilat mit 7 mol Ethylenoxid.

400 g des gemäß Beispiel 3 hergestellten Alkanolgemisches werden mit 1,5 g NaOH in einen trockenen 2 l - Autoklaven eingefüllt. Der Autoklaveninhalt wird auf 150°C erhitzt und 616 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator durch Schwefelsäurezusatz neutralisiert.
Das erhaltene Oxethylat stellt ein neutrales Tensid dar. Es hat einen Trübungspunkt von 72°C, gemessen nach DIN 53917, 1 gew.-%-ig in 10 gew.-%iger wässriger Butyldiglykollösung. Die Oberflächenspannung einer Lösung von 1 g/l der Substanz in Wasser beträgt 27,3 mN/m, gemessen nach DIN 53914.

### B. Fettalkoholethoxilat mit 3 mol Ethylenoxid.

600 g des gemäß Beispiel 3B hergestellten Alkanolgemisches werden mit 1,5 g NaOH in einen trockenen 2 l - Autoklaven eingefüllt. Der Autoklaveninhalt wird auf 150°C erhitzt und 396 g Ethylenoxid unter Druck in den Autoklaven gepreßt. Nachdem sich die gesamte Ethylenoxidmenge im Autoklaven befindet, wird der Autoklav für 30 Minuten bei 150°C gehalten. Nach dem Abkühlen wird der Katalysator durch Schwefelsäurezusatz neutralisiert.
Das erhaltene Oxethylat stellt ein neutrales Tensid dar. Es hat einen Trübungspunkt von 43,5°C, gemessen nach DIN 53917, 1 gew.-%-ig in 10 gew.-%iger wässriger Butyldiglykollösung. Die Oberflächenspannung einer Lösung von 1 g/l der Substanz in Wasser beträgt 26,1 mN/m, gemessen nach DIN 53914.

### Beispiele 5A und 5B, Herstellung von Alkyl- und Alkyletherphosphaten.

### A. Alkylphosphat.

300 g des nach Beispiel 3B hergestellten Alkoholgemisches werden in einem Rührgefäß unter Stickstoff auf 60°C erwärmt und langsam mit 125 g Polyphosphorsäure versetzt. Dabei soll die Temperatur 65°C nicht übersteigen. Gegen Ende der Zugabe wird der Ansatz auf 70°C erwärmt und eine Stunde bei dieser Temperatur weitergerührt.
Das erhaltene Produkt stellt ein anionisches Tensid dar. Eine wässrige Lösung der Substanz in Wasser hat bei einer Konzentration von 1 g/l eine Oberflächenspannung von 29,8 mN/m, gemessen nach DIN 53914.

### B. Alkyletherphosphat.

560 g des nach Beispiel 4B hergestellten Fettalkoholoxethylat-Gemisches werden in einem Rührgefäß unter Stickstoff auf 60°C erwärmt und langsam mit 92 g Polyphosphorsäure versetzt. Dabei soll die Temperatur 65°C nicht übersteigen. Gegen Ende der Zugabe wird der Ansatz auf 70°C erwärmt und eine Stunde bei dieser Temperatur weitergerührt.

Das erhaltene Produkt stellt ein anionisches Tensid dar. Eine wässrige Lösung der Substanz in Wasser hat bei einer Konzentration von 1 g/l eine Oberflächenspannung von 37,7 mN/m, gemessen nach DIN 53914.

## Patentansprüche

1. Verfahren zur Herstellung von Tensidalkoholen und Tensidalkoholethem durch Derivatisierung von Olefinen mit 10 bis 20 C-Atomen oder von Gemischen solcher Olefine und gegebenenfalls anschließende Alkoxylierung **dadurch gekennzeichnet, dass** man
a) ein C4-Olefin-Gemisch der Metathese unterwirft,
b) aus dem Metathesegemisch Olefine mit 5 bis 8 C-Atomen abtrennt,
c) die abgetrennten Olefine einzeln oder im Gemisch einer Dimerisierung zu Olefingemischen mit 10 bis 16 C-Atomen unterzieht,
d) das erhaltene Olefingemisch, gegebenenfalls nach einer Fraktionierung, der Derivatisierung zu einem Gemisch von Tensidalkoholen unterwirft und
e) gegebenenfalls die Tensidalkohole alkoxyliert.

2. Verfahren gemäß Anspruch 1, **dadurch gekennzeichnet, dass** der Verfahrensschritt a), die Metathese, in Gegenwart von molybdän-, wolfram- oder rheniumhaltigen Katalysatoren ausgeführt wird.

3. Verfahren gemäß Anspruch 1 oder 2, **dadurch gekennzeichnet, dass** man im Verfahrensschritt b) die Olefine mit 5 und 6 C-Atomen abtrennt.

4. Verfahren gemäß einem der Ansprüche 1 bis 3, **dadurch gekennzeichnet, dass** der Verfahrensschritt c), die Dimerisierung, heterogenkatalysiert durchgeführt wird.

5. Verfahren gemäß einem der Ansprüche 1 bis 4, **dadurch gekennzeichnet, dass** man einen Dimerisierungskatalysator einsetzt, der wenigstens ein Element der VIII. Nebengruppe des periodischen Systems enthält, und man die Katalysatorzusammensetzung und die Reaktionsbedingungen so wählt, daß ein Dimerengemisch erhalten wird, welches weniger als 10 Gew.-% von Verbindungen enthält, die ein Strukturelement der Formel I (Vinylidengruppe) 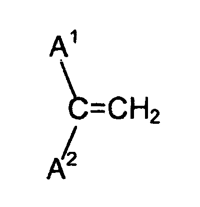 worin A¹ und A² aliphatische Kohlenwasserstoffreste sind, aufweisen.

6. Verfahren gemäß einem der Ansprüche 1 bis 5, **dadurch gekennzeichnet, dass** man im Verfahrensschritt c) die Olefine mit 5 und 6 C-Atomen einzeln oder im Gemisch dimerisiert.

7. Verfahren gemäß einem der Ansprüche 1 bis 6, **dadurch gekennzeichnet, dass** man im Verfahrensschritt c) das 3-Hexen dimerisiert.

8. Verfahren gemäß einem der Ansprüche 1 bis 7, **dadurch gekennzeichnet, dass** man die Derivatisierung (Verfahrensschritt d) durch Hydroformylierung ausführt.

9. Olefingemische, **dadurch gekennzeichnet, dass**
a) sie 10 bis 16 C-Atome und
b) einen Anteil unverzweigter Olefine von unter 25 Gew.-% aufweisen,
c) mindestens 80% der Komponenten eine Verzweigung oder zwei Verzweigungen an benachbarten C-Atomen im Bereich von 1/4 bis 3/4 der Kettenlänge der Hauptkette liegen,
d) und weniger als 10 Gew.-% von Verbindungen enthalten, die ein Strukturelement der Formel I (Vinylidengruppe) 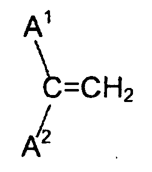
aufweisen, worin A¹ und A² aliphatische Kohlenwasserstoffreste sind.

10. Olefingemische gemäß Anspruch 9, **dadurch gekennzeichnet, dass** sie einen Anteil unverzweigter Olefine von unter 20 Gew.-%, aufweisen.

11. Olefingemische gemäß Anspruch 9 oder 10, **dadurch gekennzeichnet, dass** mindestens 80 % der Komponenten des Dimerisierungsgemisches im Bereich von 1/3 bis 2/3, der Kettenlänge ihrer Hauptkette eine Verzweigung, oder zwei Verzweigungen an benachbarten C-Atomen, aufweisen.

12. Olefingemische gemäß einem der Ansprüche 9 bis 11, **dadurch gekennzeichnet, dass** an den Verzweigungsstellen der Hauptkette überwiegend Gruppen mit (y-4) und (y-5) C-Atomen gebunden sind, wobei y die Kohlenstoffatom-Anzahl des für die Dimerisierung eingesetzten Monomers ist.

13. Olefingemische gemäß einem der Ansprüche 9 bis 12, **dadurch gekennzeichnet, dass** das Verhältnis der aliphatischen zu olefinischen Wasserstoffatomen im Bereich von H_{aliph.} : H_{olefin.} = (2*n-0,5) : 0,5 bis (2*n-1,9) : 1,9 liegt, wobei n die Kohlenstoffatom-Anzahl des bei der Dimerisierung erhaltenen Olefins ist.

14. Olefingemische gemäß einem der Ansprüche 9 bis 13, **dadurch gekennzeichnet, dass** das Verhältnis der aliphatischen zu olefinischen Wasserstoffatomen im Bereich von H_{aliph.} : H_{olefin.} = (2*n-1,0) : 1 bis (2*n-1,6): 1,6 liegt.

15. Tensidalkoholgemische **dadurch gekennzeichnet, dass**
a) sie eine Alkankette mit 10 bis 16 C-Atomen aufweisen, an die eine -OH-Gruppe oder eine -CH₂OH-Gruppe gebunden ist, und
b) einen Anteil unverzweigter Alkohole von unter 25 Gew.-% aufweisen,
c) mindestens 80% der Komponenten eine Verzweigung oder zwei Verzweigungen an benachbarten C-Atomen im Bereich von 1/4 bis 3/4 der Kettenlänge der Hauptkette liegen,
und deren Alkoxylierungsprodukte.

16. Verwendung der Tensidalkohol-Alkoxylierungsprodukte des Anspruchs 15 als nichtionische Tenside.

17. Verwendung der Tensidalkohole des Anspruchs 15 zur Herstellung von Tensiden.

18. Verwendung der Tensidalkohole des Anspruchs 15 zur Herstellung von Alkanolglycosid- und polyglycosid-Gemischen durch ein- oder mehrfache Umsetzung (Glycosidierung, Polyglycosidierung) mit Mono-, Di- oder Polysacchariden unter Ausschluß von Wasser unter Säurekatalyse oder mit O-Acetylsaccharid-haliden.

19. Verwendung der Tensidalkohole und deren Alkoxylierungsprodukte des Anspruchs 15 zur Herstellung oberflächenaktiver Sulfate durch Veresterung derselben mit Schwefelsäure oder Schwefelsäurederivaten zu sauren Alkylsulfaten oder Alkylethersulfaten.

20. Verwendung der Tensidalkohole und deren Alkoxylierungsprodukte des Anspruchs 15 zur Herstellung oberflächenaktiver Phosphate durch Veresterung derselben mit Phosphorsäure oder ihren Derivaten zu sauren Alkylphosphaten bzw. Alkyletherphosphaten.

## Claims

1. A process for the preparation of surfactant alcohols and surfactant alcohol ethers by derivatization of olefins having from 10 to 20 carbon atoms or of mixtures of such olefins and optionally subsequent alkoxylation, which comprises
a) subjecting a C₄-olefin mixture to metathesis,
b) separating off olefins having from 5 to 8 carbon atoms from the metathesis mixture,
c) subjecting the separated-off olefins individually or as a mixture to dimerization to give olefin mixtures having from 10 to 16 carbon atoms,
d) subjecting the resulting olefin mixture, optionally after fractionation, to derivatization to give a mixture of surfactant alcohols, and
e) optionally alkoxylating the surfactant alcohols.

2. A process as claimed in claim 1, wherein the process step a), the metathesis, is carried out in the presence of catalysts containing molybdenum, tungsten or rhenium.

3. A process as claimed in claim 1 or 2, which comprises, in process step b), separating off the olefins having 5 and 6 carbon atoms.

4. A process as claimed in any of claims 1 to 3, wherein process step c), the dimerization, is carried out with heterogeneous catalysis.

5. A process as claimed in any of claims 1 to 4, wherein a dimerization catalyst is used which contains at least one element from subgroup VIII of the Periodic Table of the Elements,
and the catalyst composition and the reaction conditions are chosen such that a dimer mixture is obtained which comprises less than 10% by weight of compounds which have a structural element of the formula I (vinylidene group) 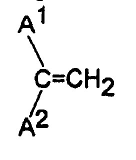 in which A¹ and A² are aliphatic hydrocarbon radicals.

6. A process as claimed in any of claims 1 to 5, wherein, in process step c) olefins having 5 and 6 carbon atoms are dimerized individually or in a mixture.

7. A process as claimed in any of claims 1 to 6, wherein, in process step c), 3-hexene is dimerized.

8. A process as claimed in any of claims 1 to 7, wherein the derivatization (process step d)) is carried out by hydroformylation.

9. An olefin mixture which has
a) 10 to 16 carbon atoms and
b) a proportion of unbranched olefins of less than 25% by weight,
c) at least 80% of the components having one branch or two branches to adjacent carbon atoms in the range from 1/4 to 3/4 of the chain length of the main chain,
d) and comprising less than 10% by weight of compounds which have a structural element of the formula I (vinylidene group) 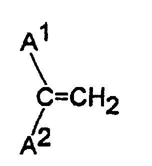
in which A¹ and A² are aliphatic hydrocarbon radicals.

10. An olefin mixture as claimed in claim 9, which has a proportion of unbranched olefins of less than 20% by weight.

11. An olefin mixture as claimed in claim 9 or 10, wherein at least 80% of the components of the dimerization mixture have, in the range from 1/3 to 2/3, of the chain length of their main chain, one branch, or two branches to adjacent carbon atoms.

12. An olefin mixture as claimed in any of claims 9 to 11, wherein, at the branching sites of the main chain, predominantly groups having (y-4) and (y-5) carbon atoms are bonded, where y is the number of carbon atoms in the monomer used for the dimerization.

13. An olefin mixture as claimed in any of claims 9 to 12, wherein the ratio of aliphatic to olefinic hydrogen atoms is in the range
H_{aliph.} : H_{olefin.} = (2*n-0.5) : 0.5 to (2*n-1.9) : 1.9, where n is the number of carbon atoms in the olefin obtained in the dimerization.

14. An olefin mixture as claimed in any of claims 9 to 13, wherein the ratio of aliphatic to olefinic hydrogen atoms is in the range
H_{aliph.} : H_{olefin.} = (2*n-1.0) : 1 to (2*n-1.6) : 1. 6.

15. A surfactant alcohol mixture which has
a) an alkane chain with 10 to 16 carbon atoms, to which an OH group or a CH₂OH group is bonded, and
b) a proportion of unbranched alcohols of less than 25% by weight,
c) at least 80% of the components having one branch or two branches to adjacent carbon atoms in the range from 1/4 to 3/4 of the chain length of the main chain,
or an alkoxylation product thereof.

16. The use of the surfactant alcohol alkoxylation products of claim 15 as nonionic surfactants.

17. The use of the surfactant alcohol of claim 15 for the preparation of surfactants.

18. The use of the surfactant alcohol of claim 15 for the preparation of alkanol glycoside and polyglycoside mixtures by single or multiple reaction (glycosylation, polyglycosylation) with mono-, di- or polysaccharides with the exclusion of water and with acid catalysis or with O-acetylsaccharide halides.

19. The use of the surfactant alcohol and its alkoxylation products of claim 15 for the preparation of surface-active sulfates by esterification thereof with sulfuric acid or sulfuric acid derivatives to give acidic alkyl sulfates or alkyl ether sulfates.

20. The use of the surfactant alcohol and its alkoxylation products of claim 15 for the preparation of surface active phosphates by esterification thereof with phosphoric acid or its derivatives to give acidic alkyl phosphates or alkyl ether phosphates.

## Revendications

1. Procédé de préparation d'alcools tensioactifs et d'alcool-éthers tensioactifs par dérivatisation d'alcools ayant de 10 à 20 atomes de carbone ou de mélange de ces oléfines, puis éventuellement alcoxylation, **caractérisé en ce que**
a) on soumet à une métathèse un mélange d'oléfines en C₄,
b) on sépare du mélange de métathèse les oléfines ayant de 5 à 8 atomes de carbone,
c) on soumet les oléfines séparées, individuellement ou en mélange, à une dimérisation pour obtenir des mélanges d'oléfines ayant de 10 à 16 atomes de carbone,
d) on soumet le mélange d'oléfines ainsi obtenues, éventuellement après un fractionnement, à une dérivatisation pour obtenir un mélange d'alcools tensioactifs, et
e) éventuellement, on soumet les alcools tensioactifs à une alcoxylation.

2. Procédé selon la revendication 1, **caractérisé en ce que** l'étape a), la métathèse, est mise en oeuvre en présence de catalyseurs contenant du molybdène, du tungstène ou du rhénium.

3. Procédé selon la revendication 1 ou 2, **caractérisé en ce que**, dans l'étape b), on sépare les oléfines ayant 5 et 6 atomes de carbone.

4. Procédé selon l'une des revendications 1 à 3, **caractérisé en ce que** l'étape c), la dimérisation, est mise en oeuvre par catalyse hétérogène.

5. Procédé selon l'une des revendications 1 à 4, **caractérisé en ce qu'**on utilise un catalyseur de dimérisation qui contient au moins un élément du VIIIème Groupe Secondaire du Tableau Périodique, et **en ce qu'**on choisit la composition du catalyseur et les conditions de réaction de façon à obtenir un mélange de dimères qui contient moins de 10 % en poids de composés comportant un élément structural de formule I (groupe vinylidène) 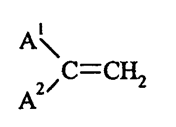 dans laquelle A¹ et A² sont des radicaux hydrocarbonés aliphatiques.

6. Procédé selon l'une des revendications 1 à 5, **caractérisé en ce qu'**on dimérise dans l'étape c) les oléfines ayant 5 et 6 atomes de carbone, à titre individuel ou en mélange.

7. Procédé selon l'une des revendications 1 à 6, **caractérisé en ce que**, dans l'étape c), on dimérise le 3-hexène.

8. Procédé selon l'une des revendications 1 à 7, **caractérisé en ce qu'**on met en oeuvre la dérivatisation (étape d) par hydroformylation.

9. Mélanges d'oléfines, **caractérisés en ce que**
a) elles ont de 10 à 16 atomes de carbone, et
b) ils présentent une proportion d'oléfines non ramifiées inférieure à 25 % en poids,
c) au moins 80 % des composants présentent une ramification ou deux ramifications sur des atomes de carbone voisins dans la zone située entre 1/4 et 3/4 de la longueur de chaîne de la chaîne principale,
d) et ils contiennent moins de 10 % en poids de composés comportant un élément structural de formule I (groupe vinylidène) 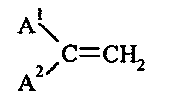
dans laquelle A¹ et A² sont des radicaux hydrocarbonés aliphatiques.

10. Mélanges d'oléfines selon la revendication 9, **caractérisés en ce qu'**ils présentent une proportion d'oléfines non ramifiées inférieure à 20 % en poids.

11. Mélanges d'oléfines selon la revendication 9 ou 10, **caractérisés en ce qu'**au moins 80 % des composants du mélange de dimérisation présentent dans la zone allant de 1/3 à 2/3 de la longueur de chaîne de leur chaîne principale une ramification ou deux ramifications sur des atomes de carbone voisins.

12. Mélanges d'oléfines selon l'une des revendications 9 à 11, **caractérisés en ce que** ce sont surtout des groupes ayant (y-4) et (y-5) atomes de carbone qui sont liés aux points de ramification de la chaîne principale, y étant le nombre d'atomes de carbone du monomère utilisé pour la dimérisation.

13. Mélanges d'oléfines selon l'une des revendications 9 à 12, **caractérisés en ce que** le rapport entre les atomes d'hydrogène aliphatiques et les atomes d'hydrogène oléfiniques est compris dans la plage Hₐₗᵢₚₕ : H_{oléfine} comprise entre (2*n-0,5):0,5 et (2*n-1,9):1,9, n étant le nombre d'atomes de carbone de l'oléfine obtenue lors de la dimérisation.

14. Mélanges d'oléfmes selon l'une des revendications 9 à 13, **caractérisés en ce que** le rapport entre les atomes d'hydrogène aliphatiques et les atomes d'hydrogène oléfiniques est compris dans la plage Hₐₗᵢₚₕ : H_{oléfine} comprise entre (2*n-1,0):1 et (2*n-1,6):1,6.

15. Mélanges d'alcools tensioactifs, **caractérisés en ce que**
a) ils comportent une chaîne alcane ayant de 10 à 16 atomes de carbone, à laquelle est lié un groupe -OH ou un groupe -CH₂OH, et
b) ils comportent une proportion d'alcools non ramifiés inférieure à 25 % en poids,
c) au moins 80 % des composants présentent une ramification ou deux ramifications sur des atomes de carbone voisins dans la zone comprise entre 1/4 et 3/4 de la longueur de chaîne de la chaîne principale,
et leurs produits d'alcoxylation.

16. Utilisation des produits d'alcoxylation des alcools tensioactifs selon la revendication 15, en tant que tensioactifs non ioniques.

17. Utilisation des alcools tensioactifs selon la revendication 15 pour préparer des tensioactifs.

18. Utilisation des alcools tensioactifs selon la revendication 15 pour préparer des mélanges d'alcanolglycosides et d'alcanolpolyglycosides, par une réaction simple ou multiple (glycosidation, polyglycosidation) avec des mono-, des di- ou des polysaccharides, à l'abri de l'eau, par catalyse acide ou avec des O-acétylsaccharide-halogénures.

19. Utilisation des alcools tensioactifs et de leurs produits d'alcoxylation selon la revendication 15 pour produire des sulfates tensioactifs par estérification de ces derniers avec de l'acide sulfurique ou des dérivés de l'acide sulfurique, pour obtenir des alkylsulfates ou des alkyléthersulfates acides.

20. Utilisation des alcools tensioactifs et de leurs produits d'alcoxylation selon la revendication 15 pour préparer des phosphates tensioactifs par estérification de ces derniers avec de l'acide phosphorique ou ses dérivés, pour donner des alkylphosphates ou des alkylétherphosphates acides.
